(19)

Europäisches Patentamt

European Patent Office

Office européen des brevets

(11)  EP 0 689 772 B1

(12)  FASCICULE DE BREVET EUROPEEN

(45) Date de publication et mention
de la délivrance du brevet:
**08.11.2000   Bulletin 2000/45**

(51) Int Cl.⁷: **A23L 1/221**, C11B 9/00

(21) Numéro de dépôt: **95401489.0**

(22) Date de dépôt: **23.06.1995**

(54) **Perles de vanilline ou d'éthylvanilline et leur procédé d'obtention**

Perlförmiges Vanillin oder Ethylvanillin und Verfahren zu deren Herstellung

Beads of vanillin or ethylvanillin and method for the preparation thereof

(84) Etats contractants désignés:
**AT BE CH DE DK ES FR GB IE IT LI NL SE**

(30) Priorité: **29.06.1994  FR 9407994**

(43) Date de publication de la demande:
**03.01.1996   Bulletin 1996/01**

(73) Titulaire: **RHODIA CHIMIE
92408 Courbevoie Cédex (FR)**

(72) Inventeurs:
 • **Brossette, Jean
F-69330 Meyzieu (FR)**
 • **Le Thiesse, Jean-Claude
F-42100 Saint-Etienne (FR)**

 • **Statiotis, Eraclis
F-38280 Villette d'Anthon (FR)**

(74) Mandataire: **Dubruc, Philippe et al
RHODIA SERVICES
Direction de la Propriété Industrielle
25, quai Paul Doumer
92408 Courbevoie Cédex (FR)**

(56) Documents cités:
**FR-A- 541 652**

 • **DATABASE WPI Week 8838 Derwent
Publications Ltd., London, GB; AN 88-266465 &
JP-A-63 097 168 (NIPPON OIL SEAL IND.) , 27
Avril 1988**

**Description**

La présente invention a pour objet une nouvelle présentation de la vanilline et/ou de l'éthylvanilline. Plus précisément, l'invention a pour objet des perles de vanilline et/ou d'éthylvanilline. L'invention se rapporte aussi à la préparation desdites perles.

La vanilline est un produit largement utilisée dans de nombreux domaines d'application en tant qu'arôme ou parfum.

Ainsi, la vanilline se trouve largement consommée dans l'industrie alimentaire mais elle a aussi des applications dans d'autres domaines tels que par exemple, la pharmacie ou la parfumerie. Il s'ensuit que c'est un produit de grande consommation.

La vanilline est actuellement disponible sur le marché sous la forme d'une poudre cristallisée. Les inconvénients qui en résultent sont la présence de fines qui entraînent des problèmes de poussièrage lors du stockage et de la manipulation de ladite poudre. De plus, on assiste souvent à des phénomènes de mottage lors de stockage prolongé.

Pour pallier les inconvénients précités, la présente invention propose une nouvelle présentation de la vanilline et de l'éthylvanilline ainsi qu'un de ses procédés d'obtention.

Plus précisément, la présente invention a pour objet des perles de vanilline et/ou d'éthylvanilline. L'invention inclut également les isomères de la vanilline et de l'éthylvanilline dénommés respectivement isovanilline et isoéthylvanilline.

Dans l'exposé de la présente invention, on entend par "perles", des particules solides à forte sphéricité.

La caractéristique du procédé de l'invention, en vue de préparer des perles de vanilline et/ou d'éthylvanilline est de faire fondre si nécessaire la vanilline et/ou l'éthylvanilline, puis de fragmenter la masse fondue en gouttelettes et solidifier les gouttelettes obtenues dans un courant gazeux de refroidissement de telle sorte qu'elles se solidifient en perles qui sont ensuite récupérées.

Une variante préférée du procédé de l'invention consiste est à faire fondre si nécessaire la vanilline et/ou l'éthylvanilline, puis à faire passer la masse fondue dans une buse de façon à former des gouttelettes, à solidifier ces dernières en les laissant tomber dans une tour à contre-courant d'un gaz froid, puis à récupérer les perles obtenues.

Les perles obtenues selon l'invention présentent des caractéristiques physico-chimiques qui leur sont propres.

Les définitions et les méthodes de détermination des caractéristiques données ci-après, sont précisées dans les exemples.

Les perles de vanilline et/ou d'éthylvanilline sont sous la forme de billes de couleur blanche. Elles présentent une taille de particules essentiellement sous forme sphérique, ayant un diamètre qui peut être choisi, grâce au procédé de l'invention, dans une large gamme. Ainsi, la taille des particules peut s'échelonner entre 200 µm et 3000 µm mais se situe, de préférence, entre 300 µm et 1000 µm. Précisons que la détermination des tailles se fait par passage sur des tamis métalliques.

Généralement, la taille des particules exprimée par le diamètre médian ($d_{50}$) varie de 500 µm à 2 000 µm, de préférence entre 500 µm et 1 000 µm. On définit le diamètre médian comme étant tel que 50 % en poids des particules ont un diamètre supérieur ou inférieur au diamètre médian.

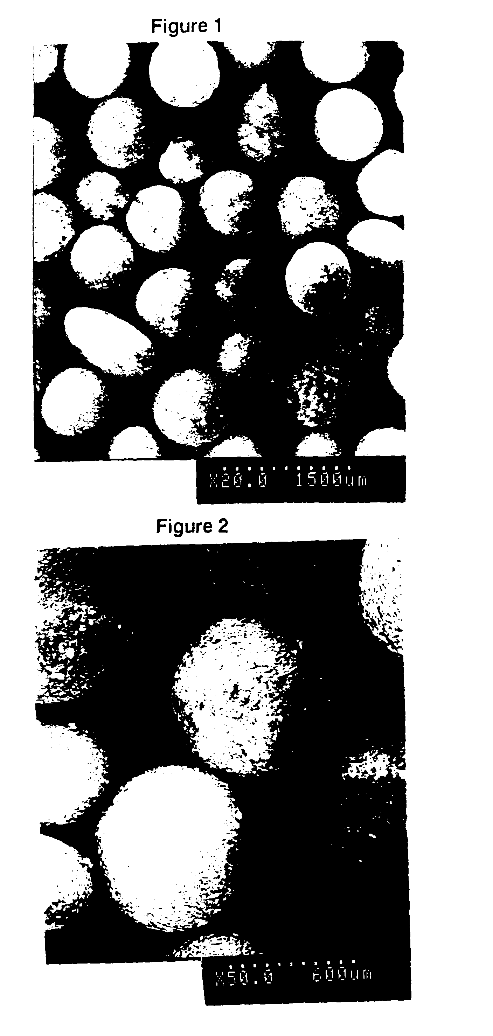

Les figures 1 et 2 représentent des photographies prises au microscope électronique à balayage (avec des grossissements respectifs de 20 et 50) qui illustrent la morphologie de type perle de la vanilline obtenue selon l'invention. On observe une répartition granulométrique uniforme sur le produit obtenu.

Les perles ont une densité qui peut être plus ou moins élevée. La densité apparente (non tassée) des perles est de préférence, d'au moins 0,7 et se situe encore plus préférentiellement entre 0,7 et 0,9. Il y a lieu de noter que les perles de l'invention présentent une densité nettement plus élevée par rapport à celle de la poudre cristallisée.

Les perles de vanilline et/ou d'éthylvanilline de l'invention ont une forme physique qui leur permettent de résister à l'attrition.

Elles présentent une cohésion adaptée à de bonnes propriétés d'écoulement.

Ainsi, l'indice d'écoulement est nettement amélioré par rapport à la poudre cristallisée ce qui est mis en évidence dans les exemples.

L'indice d'écoulement mesuré en instantané est toujours largement supérieur à 10, voire infini.

La structure originale des produits de l'invention est obtenue grâce à un procédé de fabrication parfaitement adapté.

Le procédé de l'invention de préparation de perles de vanilline et/ou d'éthylvanilline consiste à partir de vanilline et/ou d'éthylvanilline fondue, puis à fragmenter la masse fondue en gouttelettes, et à solidifier les gouttelettes obtenues dans un courant gazeux de refroidissement de telle sorte que les gouttelettes obtenues se solidifient en perles qui sont ensuite récupérées.

Dans le procédé de l'invention, on met en oeuvre de la vanilline et/ou l'éthylvanilline fondue.

On peut envisager d'alimenter directement la vanilline et/ou l'éthylvanilline fondue provenant d'une ligne de fabrication.

Il est également possible de prévoir une étape de procédé de l'invention qui consiste à faire fondre la vanilline et/ou l'éthylvanilline si l'on dispose d'une forme poudre. A cet effet, on chauffe le produit à sa température de fusion. De préférence, on porte le produit à une température légèrement supérieure à sa température de fusion, de préférence supérieure à au plus 5°C par rapport à son point de fusion. Pour la vanilline, la température à laquelle est portée celle-ci, est choisie entre 81°C et 86°C. En ce qui concerne l'éthylvanilline, la température est choisie entre 74°C et 79°C.

Il est possible de mettre en oeuvre de la vanilline additivée d'éthylvanilline et inversement. On met en oeuvre de préférence, moins de 40 %, et encore plus préférentiellement moins de 20 % de l'autre constituant. La température à laquelle sera portée le mélange pourqu'il fonde sera aisément déterminée par l'Homme du métier, par de simples opérations d'exécution.

On ne sortira pas également du cadre de la présente invention, à introduire également des additifs solubles ou en fine suspension dans la vanilline et/ou l'éthylvanilline.

Cette opération de mise en fusion du ou des produits est effectuée, généralement sous agitation. Un mode de réalisation préférée de l'invention, consiste à effectuer cette opération sous atmosphère de gaz inertes qui peut être un gaz rare mais pour des raisons économiques, préférentiellement l'azote.

Dans une étape suivante, on transforme la masse fondue, en gouttelettes. Cette opération peut être réalisée au moyen de tout dispositif de fragmentation, par exemple, par une buse plate à orifice(s) circulaire(s).

Un mode de réalisation préférentielle de l'invention consiste à former les gouttelettes par passage de la masse fondue au travers d'un orifice et tout particulièrement par passage au travers d'une buse.

L'opération suivante est d'assurer la solidification des gouttelettes en perles, par contact avec un gaz froid dont la température est choisie entre -50°C et 0°C, de préférence, entre -30°C et -10°C.

Le gaz froid est un gaz quelconque dans la mesure où il est inerte vis-à-vis de la vanilline et/ou l'éthylvanilline. On choisit de préférence, l'azote ou l'air appauvri en oxygène (par exemple à 10 %).

D'une manière préférentielle, on envoie le courant gazeux froid, à contre-courant du flux de matière.

Le temps de séjour qui est la durée entre la formation de la gouttelette à la sortie de la buse et son arrivée dans le système de récupération se situe avantageusement, entre 1 et 10 secondes et plus préférentiellement entre 3 et 5 secondes.

Une façon d'obtenir le temps de séjour désiré, est de laisser tomber les gouttelettes dans une tour à contre-courant d'un gaz froid tel que précité.

En fin de réaction, on récupère les perles par tout moyen connu, par exemple, par gravité dans un bac de récupération ou selon la technique de lit fluide.

En ce qui concerne l'appareillage utilisé pour mettre en oeuvre le procédé de l'invention, il est composé de deux ensembles : un premier ensemble de mise en forme des perles et un deuxième ensemble de récupération des perles.

Le premier ensemble comprend un bac de stockage de préférence agité lorsque la vanilline et/ou l'éthylvanilline provient d'une ligne de fabrication ou bien un fondoir permettant de fondre la vanilline et/ou l'éthylvanilline et une enceinte qui est généralement, une tour comprenant dans sa partie supérieure, un dispositif de fragmentation en gouttelettes, de préférence une buse et équipée dans sa partie inférieure d'une ou plusieurs arrivées d'un courant gazeux froid transformant ainsi le bas de la tour en une tour de refroidissement.

La hauteur de la tour peut varier très largement par exemple, entre 6 et 40 mètres, selon la taille de l'installation. Il est à noter que la borne limite supérieure ne présente aucun caractère critique.

La vanilline et/ou l'éthylvanilline est introduite par une trémie à double vis, dans un fondoir qui est un réacteur équipé d'un système permettant de réguler la température, par exemple, une double enveloppe, afin de maintenir la vanilline et/ou l'éthylvanilline à l'état fondu.

La buse utilisée peut être une buse à un seul trou ou une buse multi-trous avec un nombre de trous qui peut varier entre 1 et 3000 trous, et de préférence, entre 1 et 100 trous.

On peut utiliser un système comprenant plusieurs buses, par exemple, 2 buses de préférence amovibles, en parallèle.

Le diamètre des perforations de la buse est fonction de la taille des perles désirées. Il peut être de 50 à 2000 μm mais il est choisi, de préférence, entre 200 μm et 600 μm.

La taille de la perforation est toujours inférieure à la taille de la perle obtenue. Ainsi, on utilise une buse présentant des perforations d'environ 200 μm pour obtenir des perles présentant un diamètre médian de 500 μm.

La buse utilisée peut être une buse statique mais il est possible de faire appel à une buse soumise à un système de vibration électrique de haute fréquence, par exemple, de 100 à 10000 hertz. Ce dispositif permet d'obtenir des goutelettes de taille parfaitement calibrée.

La masse fondue arrive dans la buse de préférence, par une surpression qui est assurée par un courant gazeux, de préférence, un courant d'azote. La surpression par rapport à la pression atmosphérique est de 5 à 500 %.

La buse est maintenue à la température de fusion du produit.

Au niveau de la buse, il est possible mais non indispensable d'établir un courant gazeux, de préférence un co-courant d'azote avec le jet sortant de la buse. Ce courant gazeux a, de préférence, une température comprise entre la température ambiante et 80°C. Le présence de ce co-courant gazeux permet d'obtenir une meilleure régularité de la dimension des perles et évite la coalescence des gouttes.

Dans la partie supérieure de la tour, il peut y avoir sur la paroi interne de la tour, présence de chicanes et de grilles permettant une distribution homogène du flux gazeux.

Dans le bas de la tour, on introduit un courant de gaz froid, de préférence, d'azote ou d'air appauvri en oxygène. Ce courant gazeux froid assure la solidification des gouttelettes en perles. Il a de préférence une température comprise entre -50°C et 0°C, de préférence, comprise entre -30°C et -10°C.

Le courant gazeux froid sort, de préférence, de la tour, en dessous de la buse, à une distance représentant environ un dixième de la hauteur totale de la zone de refroidissement.

Le système de récupération des perles, en bas de la tour, ne présente pas de caractéristique critique. Il peut s'agir d'un bac de récupération ou bien d'un dispositif permettant d'assurer la fluidisation du lit de particules. Il est constitué par un bac, de préférence cylindrique, comportant dans sa partie inférieure, une grille au travers de laquelle est envoyé un courant gazeux, de préférence, d'azote ou d'air appauvri en oxygène. Le débit gazeux, qui dépend de la taille des particules, doit être tel qu'il maintient les particules en suspension. On précise, à titre d'exemple, qu'il est de 5 à 30 m$^3$/h pour un diamètre de lit fluide de 80 mm.

Dans cette partie de l'appareil, le refroidissement peut être éventuellement poursuivi.

Le dispositif de fluidisation dispose d'une sortie permettant l'évacuation des perles.

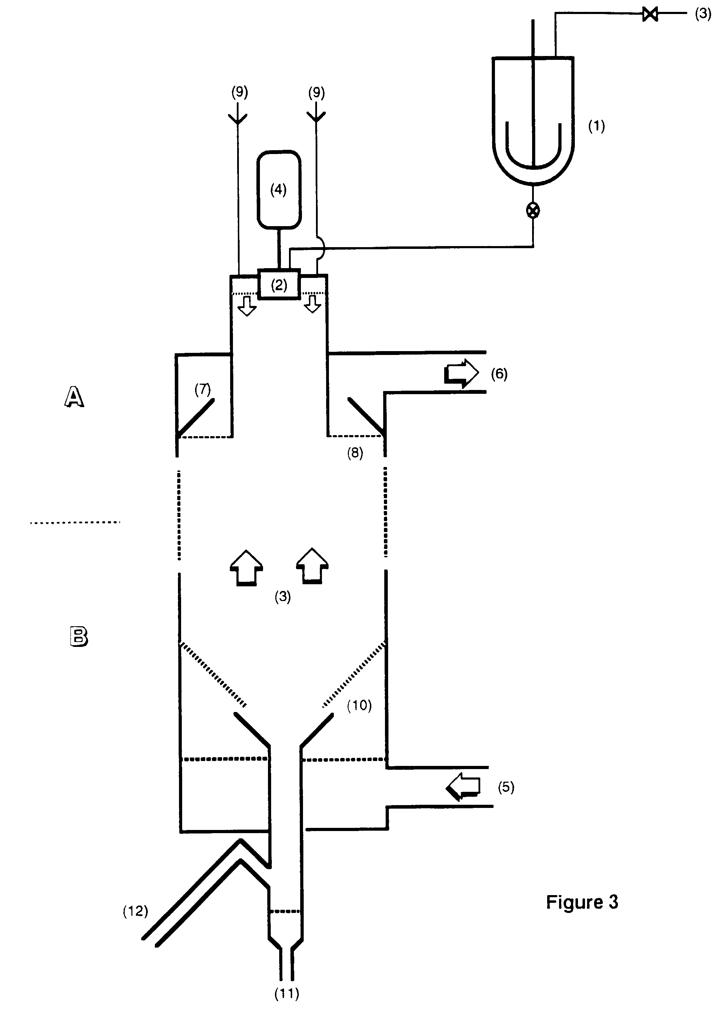

Un mode de réalisation pratique de l'invention est illustré par le dessin annexé sous forme de figure 3.

La figure 3 est une vue latérale schématique d'un appareil adapté à la mise en oeuvre de l'invention.

L'appareil utilisé est constitué de deux parties : la partie supérieure ou tour de prilling (A) et la partie inférieure qui schématise un dispositif de fluidisation (B).

La poudre de vanilline (et/ou d'éthylvanilline) est introduite dans le pot (1) où elle est fondue avant d'être acheminée vers la buse (2). Pour cela, de l'azote (3) est admis en surpression dans le bac à réserve (1).

La tour d'une hauteur de 8 mètres, comprend dans sa partie supérieure une buse (2) solidaire d'un vibrateur (4) et est équipée dans sa partie inférieure d'une arrivée d'un courant d'air froid appauvri en oxygène (5).

L'air de refroidissement introduit en (5) ressort de la tour au point (6) en dessous de la buse (2).

Dans la partie supérieure de la tour, des chicanes (7) ainsi qu'une grille (8) de forme annulaire assurent une distribution homogène du flux gazeux dans la tour. Un flux d'azote (9) chaud ayant une température comprise entre 20°C et 80°C, de préférence entre 60°C et 80°C, est distribué à co-courant autour de la buse (2).

Dans la partie inférieure de la tour, une grille de forme tronconique (10) permet de collecter les perles solidifiées dans un dispositif de fluidisation comprenant une arrivée d'azote en (11) et une sortie (12) permettant l'évacuation en continu des perles obtenues.

On donne ci-après, des exemples de réalisation pratique de l'invention.

Avant de détailler les exemples, on précise les méthodes utilisées pour la détermination des différentes caractéristiques des produits obtenus.

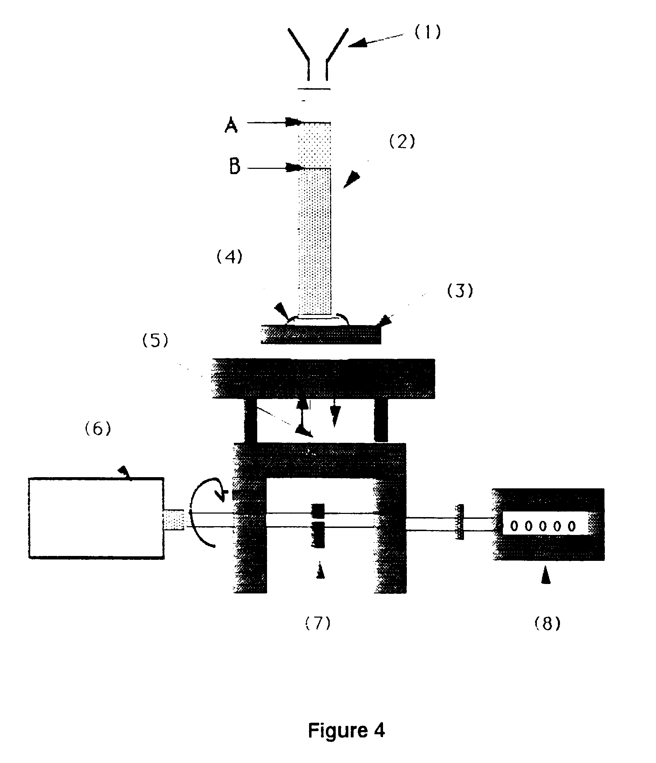

- la densité apparente tassée et non tassée :
    On la mesure sur un appareil illustré par la figure 4.
    On commence par peser l'éprouvette vide (2).
    On introduit dans l'éprouvette (2) la poudre à mesurer à l'aide de l'entonnoir (1), de manière à ce que le haut du lit de poudre vienne au ras du haut de l'éprouvette jaugée à 250 cm$^3$ (niveau A).
    On détermine la masse de la poudre par pesée de l'éprouvette pleine.
    On assujettit l'éprouvette sur le support (3) par l'intermédiaire de pinces (4).
    On met à zéro le compteur (8) qui totalise le nombre de coups imposés au fond de l'éprouvette.
    L'éprouvette est soumise à des chocs verticaux appliqués à sa base par l'intermédiaire d'un marteau (5) actionné par un moteur (6) via une came (7). On arrête l'opération lorsque le volume obtenu est constant (niveau B).
    On enregistre l'évolution du volume apparent lu sur les graduations de l'éprouvette en fonction du nombre de coups appliqués à l'aide d'un marteau.
    On obtient une courbe expérimentale de tassement.
    Volume apparent = f (nombre de coups) que l'on transforme en une courbe densité apparente = f (nombre de coups).
    On détermine la densité apparente selon la relation :

$$\text{densité apparente} = \frac{\text{masse de la poudre introduite (g)}}{\text{volume apparent (cm}^3)}$$

**-** l'indice d'écoulement :

Les propriétés d'écoulement des produits de l'invention sont mesurées en cellule de Jenike, selon la méthode explicitée par L. SVAROSVSKY dans Powder Testing Guide : Methods of measuring the physical properties of bulk powders - Elsevier Applied Science pp. 49-52 (1987).

On rappellera qu'une poudre s'écoule d'autant plus facilement que l'indice d'écoulement est plus élevé et qu'une poudre est considérée comme très cohésive lorsqu'elle a un indice d'écoulement de 2 et beaucoup moins, avec un indice inférieure à 4. On considère qu'une poudre s'écoule bien à partir d'un indice égal à 4.

**-** la vitesse de dissolution dans différents milieux :

Dans une éprouvette, on introduit à 25°C sous agitation (200 tours/minute), x g de perles dans un litre d'un liquide dont la nature est précisée ci-après.

On suit l'évolution de l'absorbance de la solution d'un rayonnement UV (spectophotomètre) en fonction du temps. Le temps de dissolution exprimé en secondes est obtenu lorsque l'absorbance se stabilise à sa valeur finale.

Les exemples qui suivent, illustrent l'invention, sans pour autant la limiter.

EXEMPLES :

On définit, ci-après, le protocole opératoire qui sera repris dans les exemples suivants.

Les perles de vanilline sont préparées dans un appareillage tel que schématisé par la figure 3.

La buse, soumise a des vibrations, présente des caractéristiques précisées dans les exemples suivants.

On part de 500 g de vanilline cristallisée.

On introduit la poudre de vanilline dans le pot (1). La surpression de l'azote en (3) varie selon les exemples : elle est voisine de 0,1 Bar.

On fond la vanilline dans le fondoir par chauffage à l'aide d'eau chaude circulant dans la double enveloppe. La température du produit est de 84°C en (1) et la température en (2), en sortie de buse est mentionnée dans le tableau récapitulatif donné dans chaque exemple. Le débit du produit à la sortie de la buse en (2) est également mentionné dans ledit tableau.

On introduit en (5) de l'air de refroidissement à un débit de 850 m$^3$/h, soit une vitesse dans la tour de 0,6 m/s. L'air ressort en (6).

Les températures définies ci-après, sont également précisées dans le tableau récapitulatif donné dans chaque exemple:

**-** température de l'air à l'entrée de la tour (5),
**-** température de l'air à la sortie de la tour en (6),
**-** température de l'air de fluidisation en (11).

Les perles obtenues sont collectées en (10) et évacuées en (12).

Exemple 1 :

Les perles de vanilline sont préparées dans un appareillage tel que schématisé par la figure 3, comportant une buse à 1 trou, ayant un diamètre de trous de 400 μm. Le rapport L/D est de 3 ; L représentant la longueur de l'orifice et D le diamètre de l'orifice.

Le procédé mis en oeuvre est tel que décrit ci-dessus : les conditions de marche étant précisées dans le tableau I suivant :

Tableau I

| | |
|---|---|
| Fréquence de vibration de la buse (2) en hertz | 1020 |
| Surpression de l'azote (3) en bars | 0,10 |
| Température du produit fondu en (2) en °C | 83,5 |
| Débit du produit à la sortie de la buse (2) en kg/h | 0,95 |
| Température de l'air à l'entrée de la tour (5) en °C | - 30 |
| Température de l'air à la sortie de la tour (6) en °C | - 20 |
| Température de l'air du lit fluide (11) en °C | - 35 |

Après 31 minutes de fonctionnement, on récupère 500 g de perles ayant un diamètre médian ($d_{50}$) de 900 $\mu$m.

Exemple 2 :

Les perles de vanilline sont préparées dans un appareillage tel que schématisé par la figure 3, comportant une buse à 1 trou, ayant un diamètre de trous de 250 $\mu$m. Le rapport L/D est de 3 ; L représentant la longueur de l'orifice et D le diamètre de l'orifice.
Le procédé mis en oeuvre est tel que décrit ci-dessus : les conditions de marche étant précisées dans le tableau II suivant :

Tableau II

| | |
|---|---|
| Fréquence de vibration de la buse (2) en hertz | 2000 |
| Surpression de l'azote (3) en bars | 0,15 |
| Température du produit fondu en (2) en °C | 81,5 |
| Débit du produit à la sortie de la buse (2) en kg/h | 0,46 |
| Température de l'air à l'entrée de la tour (5) en °C | - 35 |
| Température de l'air à la sortie de la tour (6) en °C | - 25 |
| Température de l'air du lit fluide (11) en °C | - 55 |

On récupère 500 g de perles ayant un diamètre médian ($d_{50}$) de 500 $\mu$m.

Exemple 3 :

Les perles de vanilline sont préparées dans un appareillage tel que schématisé par la figure 3, comportant une buse à 1 trou, ayant un diamètre de trous de 250 $\mu$m. Le rapport L/D est de 3 ; L représentant la longueur de l'orifice et D le diamètre de l'orifice.
Le procédé mis en oeuvre est tel que décrit ci-dessus : les conditions de marche étant précisées dans le tableau III suivant :

Tableau III

| | |
|---|---|
| Fréquence de vibration de la buse (2) en hertz | 848 |
| Surpression de l'azote (3) en bars | 0,14 |
| Température du produit fondu en (2) en °C | 81,5 |
| Débit du produit à la sortie de la buse (2) en kg/h | 0,43 |
| Température de l'air à l'entrée de la tour (5) en °C | - 10 |
| Température de l'air à la sortie de la tour (6) en °C | - 5 |
| Température de l'air du lit fluide (11) en °C | - 55 |

On récupère 500 g de perles ayant un diamètre médian ($d_{50}$) de 640 $\mu$m.

Exemple 4 :

Les perles de vanilline sont préparées dans un appareillage tel que schématisé par la figure 3, comportant une buse à 1 trou, ayant un diamètre de trous de 200 $\mu$m. Le rapport L/D est de 3; L représentant la longueur de l'orifice et D le diamètre de l'orifice.
Le procédé mis en oeuvre est tel que décrit ci-dessus : les conditions de marche étant précisées dans le tableau IV suivant :

Tableau IV

| | |
|---|---|
| Fréquence de vibration de la buse (2) en hertz | 1050 |

Tableau IV   (suite)

| | |
|---|---|
| Surpression de l'azote (3) en bars | 0,17 |
| Température du produit fondu en (2) en °C | 82 |
| Débit du produit à la sortie de la buse (2) en kg/h | 0,27 |
| Température de l'air à l'entrée de la tour (5) en °C | - 20 |
| Température de l'air à la sortie de la tour (6) en °C | - 15 |
| Température de l'air du lit fluide (11) en °C | - 55 |

On récupère 500 g de perles ayant un diamètre médian ($d_{50}$) de 520 µm.

Exemple 5 :

1 - Les perles de vanilline sont préparées dans un appareillage tel que schématisé par la figure 3, comportant une buse à 7 trous, ayant un diamètre de trous de 350 µm. Le rapport L/D est de 5 ; L représentant la longueur de l'orifice et D le diamètre de l'orifice.
Le procédé mis en oeuvre est tel que décrit ci-dessus : les conditions de marche étant précisées dans le tableau V suivant :

Tableau V

| | |
|---|---|
| Fréquence de vibration de la buse (2) en hertz | 966 |
| Surpression de l'azote (3) en bars | 0,17 |
| Température du produit fondu en (2) en °C | 83 |
| Débit du produit à la sortie de la buse (2) en kg/h | 4,8 |
| Température de l'air à l'entrée de la tour (5) en °C | - 50 |
| Température de l'air à la sortie de la tour (6) en °C | - 30 |
| Température de l'air du lit fluide (11) en °C | - 30 |

Après 30 minutes de fonctionnement, on récupère 2400 g de perles ayant un diamètre médian ($d_{50}$) de 710 µm.

2 - Les caractéristiques physico-chimiques des perles obtenues sont les suivantes :
La densité apparente non tassée est de 0,78 et la densité apparente tassée est de 0,81.
L'indice de coulabilité mesuré en cellule de Jenike est infini en instantané et de 3, après 1 jour de stockage sous consolidation. A titre de comparaison, on précise que ses valeurs sont respectivement de 3 et 0,6, pour une poudre de vanilline cristallisée du commerce.
Les vitesses de dissolution mesurées selon le test donné ci-dessus, dans différents milieux sont précisées dans le tableau suivant VI :

Tableau VI

| Quantité de perles introduites $x_g$ | Milieu de dissolution à 25°C | Temps de dissolution des perles à raison de | | | |
|---|---|---|---|---|---|
| | | 50 % en poids | 80 % en poids | 90 % en poids | 100 % en poids |
| 0,5 | solution aqueuse de sucrose à 10 % en poids | 45 s | 1 min 10 | 1 min 30 | 4 min |
| 0,5 | solution aqueuse à pH = 2 acidifiée par HCl | 1 min | 1 min 30 | 2 min | 3 min 30 |

Tableau VI   (suite)

| Quantité de perles introduites $x_g$ | Milieu de dissolution à 25°C | Temps de dissolution des perles à raison de | | | |
|---|---|---|---|---|---|
| | | 50 % en poids | 80 % en poids | 90 % en poids | 100 % en poids |
| 200 | monopropylèneglycol | 2 min | 4 min | 6 min | 10 min |
| 200 | éthanol | | | | 2 min |

**Revendications**

1.  Perles de vanilline et/ou d'éthylvanilline et isomères.

2.  Perles selon la revendication 1 caractérisées par le fait qu'elles ont une taille s'échelonnant entre 200 µm et 3000 µm mais se situant, de préférence, entre 300 µm et 1000 µm.

3.  Perles selon l'une des revendications 1 et 2 caractérisées par le fait qu'elles ont une taille exprimée par le diamètre médian ($d_{50}$) variant de 500 µm à 2000 µm, et de préférence entre 500 µm et 1000 µm.

4.  Perles selon l'une des revendications 1 à 3 caractérisées par le fait qu'elles ont une densité apparente (non tassée) d'au moins 0,7 et plus préférentiellement entre 0,7 et 0,9.

5.  Perles selon l'une des revendications 1 à 4 caractérisées par le fait qu'elles présentent un indice d'écoulement instantané supérieur à 10 voire infini.

6.  Procédé de préparation de perles de vanilline et/ou d'éthylvanilline et isomères décrites dans l'une des revendications 1 à 5 caractérisé par le fait qu'il consiste à faire fondre si nécessaire la vanilline et/ou l'éthylvanilline ou leurs isomères, puis à fragmenter la masse fondue en gouttelettes et à solidifier les gouttelettes obtenues dans un courant gazeux de refroidissement de telle sorte que les gouttelettes obtenues se solidifient en perles qui sont ensuite récupérées.

7.  Procédé selon la revendication 6 caractérisé par le fait qu'il consiste à faire passer la masse fondue de vanilline et/ou d'éthylvanilline ou leurs isomères dans une buse de façon à former des gouttelettes, à solidifier ces dernières en les laissant tomber dans une tour à contre-courant d'un gaz froid puis à récupérer les perles obtenues.

8.  Procédé selon l'une des revendications 6 et 7 caractérisé par le fait que l'on fait fondre la vanilline et/ou l'éthylvanilline ou leurs isomères à sa température de fusion, de préférence à une température supérieure à au plus 5°C par rapport à son point de fusion.

9.  Procédé selon la revendication 7 caractérisé par le fait que la buse utilisée est une buse à un seul trou ou une buse multi-trous avec un nombre de trous variant entre 1 et 3000 trous, de préférence entre 1 et 100 trous.

10. Procédé selon la revendication 7 caractérisé par le fait que la buse utilisée comporte des perforations dont le diamètre varie entre 50 à 2000 µm et se situe, de préférence, entre 200 et 600 µm.

11. Procédé selon l'une des revendications 9 et 10 caractérisé par le fait que la buse utilisée est une buse statique mais de préférence une buse soumise à un système de vibration électrique de haute fréquence, de préférence, de 100 à 10000 Hertz.

12. Procédé selon l'une des revendications 6 à 11 caractérisé par le fait que les gouttelettes sont mises en contact avec un gaz froid, de préférence d'azote ou d'air appauvri en oxygène dont la température est choisie entre -50°C et 0°C, de préférence, entre -30°C et -10°C.

13. Procédé selon l'une des revendications 6 à 12 caractérisé par le fait que le temps de séjour de la gouttelette à la sortie de la buse et son arrivée dans le système de récupération se situe avantageusement, entre 1 et 10 secondes et plus préférentiellement entre 3 et 5 secondes.

**14.** Procédé selon l'une des revendications 6 à 13 caractérisé par le fait que l'on récupère les perles par tout moyen connu, de préférence selon la technique de lit fluide.

**Claims**

**1.** Beads of vanillin and/or ethylvanillin and isomers thereof.

**2.** Beads according to claim 1, characterised in that they are between 200 µm and 3000 µm in size, preferably between 300 µm and 1000 µm.

**3.** Beads according to claim 1 or claim 2, characterised in that the size, expressed as the median diameter ($d_{50}$), is between 500 µm and 2000 µm, preferably between 500 µm and 1000 µm.

**4.** Beads according to any one of claims 1 to 3, characterised in that the (loose packed) bulk density is at least 0.7, preferably between 0.7 and 0.9.

**5.** Beads according to any one of claims 1 to 4, characterised in that the instantaneous discharge coefficient is higher than 10, possibly infinity.

**6.** A process for the preparation of beads of vanillin and/or ethylvanillin and isomers thereof as defined in any one of claims 1 to 5, characterised in that it consists of melting the vanillin and/or ethylvanillin if necessary, then fragmenting the molten mass into droplets and solidifying the droplets obtained in a gaseous cooling current so that the droplets solidify into beads which are then recovered.

**7.** A process according to claim 6, characterised in that it consists of passing the molten mass of vanillin and/or ethylvanillin through a nozzle to form droplets, solidifying the latter by allowing them to fall in a tower against a counter-current of a cold gas, then recovering the beads obtained.

**8.** A process according to claim 6 or claim 7, characterised in that the vanillin and/or ethylvanillin is melted at its melting point, preferably at a temperature of at most 5°C above its melting point.

**9.** A process according to claim 7, characterised in that the nozzle has a single hole or is a multihole nozzle with between 1 and 3000 holes, preferably between 1 and 100 holes.

**10.** A process according to claim 7, characterised in that the nozzle includes perforations with a diameter of between 50 and 2000 µm, preferably between 200 and 600 µm.

**11.** A process according to claim 9 or claim 10, characterised in that the nozzle is a static nozzle but is preferably a nozzle provided with a high frequency electrical vibration system, preferably 100 to 10000 Hertz.

**12.** A process according to any one of claims 6 to 11, characterised in that the droplets are brought into contact with a cold gas, preferably nitrogen or oxygen-depleted air, at a temperature of between -50°C and 0°C, preferably between -30°C and -10°C.

**13.** A process according to any one of claims 6 to 12, characterised in that the residence time for the droplet between the nozzle outlet and its arrival in the recovery system is advantageously between 1 and 10 seconds, preferably between 3 and 5 seconds.

**14.** A process according to any one of claims 6 to 13, characterised in that the beads are recovered using any known means, preferably using a fluid bed technique.

**Patentansprüche**

**1.** Perlen aus Vanillin und/oder Ethylvanillin und deren Isomeren.

**2.** Perlen nach Anspruch 1, dadurch gekennzeichnet, daß ihre Größe zwischen 200 µm und 3.000 µm, aber vorzugsweise zwischen 300 µm und 1.000 µm liegt.

3.  Perlen nach Anspruch 1 oder 2, dadurch gekennzeichnet, daß ihre als mittlerer Durchmesser ($d_{50}$) definierte Größe zwischen 500 µm und 2.000 µm, vorzugsweise zwischen 500 µm und 1.000 µm, schwankt.

4.  Perlen nach einem der Ansprüche 1 bis 3, dadurch gekennzeichnet, daß sie eine Schüttdichte (nicht eingerüttelt) von mindestens 0,7 und vorzugsweise zwischen 0,7 und 0,9 haben.

5.  Perlen nach einem der Ansprüche 1 bis 4, dadurch gekennzeichnet, daß sie ein momentanes Abflußverhältnis von mehr als 10 aufweisen, das sogar unendlich groß sein kann.

6.  Verfahren zur Herstellung von in den Ansprüchen 1 bis 5 beschriebenen Perlen aus Vanillin und/oder Ethylvanillin und deren Isomeren, dadurch gekennzeichnet, daß es darin besteht, das Vanillin und/oder Ethylvanillin, falls erforderlich, zu schmelzen, danach die Schmelzmasse in Tröpfchen zu zerteilen und die erhaltenen Tröpfchen in einem kühlenden Gasstrom erstarren zu lassen, so daß die erhaltenen Tröpfchen perlenförmig erstarren und diese anschließend aufgefangen werden.

7.  Verfahren nach Anspruch 6, dadurch gekennzeichnet, daß es darin besteht, die Schmelze des Vanillins und/oder Ethylvanillins durch eine Düse zu leiten, so daß Tröpfchen gebildet werden, letztere erstarren zu lassen, indem sie in einen Turm mit Gegenstrom eines kalten Gases fallen gelassen werden, und anschließend die erhaltenen Perlen aufzufangen.

8.  Verfahren nach Anspruch 6 oder 7, dadurch gekennzeichnet, daß man das Vanillin und/oder Ethylvanillin bei seiner Schmelztemperatur, vorzugsweise bei einer höchstens 5 °C über seinem Schmelzpunkt liegenden Temperatur, schmilzt.

9.  Verfahren nach Anspruch 7, dadurch gekennzeichnet, daß die verwendete Düse eine Düse mit einer einzelnen Öffnung oder eine Düse mit mehreren Öffnungen ist und die Anzahl der Öffnungen zwischen 1 und 3.000, vorzugsweise zwischen 1 und 100, liegt.

10. Verfahren nach Anspruch 7, dadurch gekennzeichnet, daß die verwendete Düse Perforationen aufweist, deren Durchmesser zwischen 50 µm und 2.000 µm, vorzugsweise zwischen 200 µm und 600 µm, liegt.

11. Verfahren nach Anspruch 9 oder 10, dadurch gekennzeichnet, daß die verwendete Düse eine unbewegliche Düse ist, die einem elektrischen Vibrationssystem ausgesetzt ist, das mit einer hohen Frequenz arbeitet, die vorzugsweise zwischen 100 und 10.000 Hertz liegt.

12. Verfahren nach einem der Ansprüche 6 bis 11, dadurch gekennzeichnet, daß die Tröpfchen einem kalten Gas ausgesetzt sind, das vorzugsweise Stickstoff oder sauerstoffarme Luft ist und dessen Temperatur zwischen -50 °C und 0 °C, vorzugsweise zwischen -30 °C und -10 °C, gewählt ist.

13. Verfahren nach einem der Ansprüche 6 bis 12, dadurch gekennzeichnet, daß die Verweilzeit des Tröpfchens zwischen Austritt an der Düse und seiner Ankunft in der Auffangvorrichtung vorteilhafterweise zwischen 1 und 10 Sekunden, vorzugsweise zwischen 3 und 5 Sekunden, liegt.

14. Verfahren nach einem der Ansprüche 6 bis 13, dadurch gekennzeichnet, daß man die Perlen unter Zuhilfenahme aller bekannten Mittel, vorzugsweise unter Verwendung der Fließbettechnik, auffängt.

## Figure 1

## Figure 2

Figure 3

Figure 4